# EUROPEAN PATENT APPLICATION

(11) **EP 3 545 819 A1**
(43) Date of publication of application: **02.10.2019**
(21) Application number: 17873182.4
(22) Date of filing: 24.10.2017
(51) Int. Cl.: A61B 5/00, G01N 21/64

(54) **PORTABLE MEASURING INSTRUMENT**

(30) Priority: 24.11.2016 JP 2016227918
(71) Applicant: NS Materials Inc., Chikushino City Fukuoka 818-0042 (JP)
(72) Inventor: KANAUMI, Eiichi, Chikushino-city Fukuoka 818-0041 (JP); OGAWA, Tomoyuki, Chikushino-city Fukuoka 818-0041 (JP); MIYANAGA, Akiharu, Chikushino-city Fukuoka 818-0042 (JP)
(74) Representative: Kraus & Weisert Patentanwälte PartGmbB
(86) International application number: PCT/JP2017/038390
(87) International publication number: WO 2018/096864

(57) **Abstract**

Provided is a measuring instrument which is portable while enabling the measurement of the accumulation level of fluorescent substances accumulated in the skin. The portable measuring instrument (10) is provided with accumulation level measuring units (31, 32, 71) measuring the accumulation level of fluorescent substances accumulated per unit area in the skin (30), a display unit (62) displaying the measured accumulation level, and a case (20) which stores or supports the accumulation level measuring units and the display unit (62) and which is to be held by a user.

## Description

### Technical Field

The present invention relates to a portable measuring instrument measuring the accumulation level of fluorescent substances in the skin and the elastic modulus of the skin.

### Background Art

AGEs (Advanced Glycation End Products) are regarded as substances generated by saccharification of proteins and causing the advancement of aging. The AGEs are fluorescent substances and emit fluorescence having a 440 nm wavelength when irradiated with excitation light having a 370 nm wavelength. A measuring instrument measuring the AGEs (Advanced Glycation End Products) utilizing such a property of the AGEs is known heretofore.

The measuring instrument of this type has an irradiation unit emitting excitation light and a light receiving unit receiving fluorescence generated from the AGEs accumulated in the skin irradiated with the excitation light and measures the accumulation level of the AGEs based on the intensity of the received fluorescence. The measuring instrument of this type is a relatively large-sized device having a base on which an arm or a hand is placed and the measurement place is fixed to, for example, an arm or a finger. The measurement is performed by irradiating an arm or a finger with excitation light in a state where the arm or a hand is placed on the base. Patent Document 1 describes a device having a measuring head having an irradiation unit and a light receiving unit and an operation box electrically connected to the measuring head as an example of such a measuring instrument of the AGEs.

### Citation List

### Patent Literature

Patent Document 1: Japanese Unexamined Patent Application Publication No. 2016-112375

### Summary of Invention

### Technical Problem

A conventional instrument for measuring the AGEs is difficult to measure the accumulation level of the AGEs in the skin of a face, for example, because the measurement place is fixed to an arm or a finger. On the other hand, in the measuring instrument described in Patent Document 1, the measuring head is separated from the operation box, and therefore the measurement place is not fixed but it is inconvenient to use the measuring instrument while carrying the measuring instrument.

The present invention has been made in view of the above-described circumstances. It is an object of the present invention to provide a measuring instrument which is portable while enabling the measurement of the accumulation level of fluorescent substances accumulated in the skin.

### Solution to Problem

(1) A portable measuring instrument according to the present invention is provided with an accumulation level measuring unit measuring the accumulation level of fluorescent substances accumulated per unit area in a skin, a display unit displaying the measured accumulation level, and a case which stores or supports the accumulation level measuring unit and the display unit and which is to be held by a user, in which the accumulation level measuring unit includes an irradiation unit emitting excitation light toward the skin, a light receiving unit receiving fluorescence generated from the fluorescent substances in the skin irradiated with the excitation light and detecting the intensity of the received fluorescence, and an accumulation level calculation unit calculating the accumulation level of the fluorescent substances based on the detected intensity.
   According to the above-described configuration, the accumulation level measuring unit measuring the accumulation level of the fluorescent substances is stored in or supported by the case to be held by a user. Therefore, the portable measuring instrument of the above-described configuration is portable while enabling the measurement of the accumulation level of the fluorescent substances accumulated in the skin.
(2) Preferably, the portable measuring instrument is further provided with an elastic modulus measuring unit measuring the elastic modulus of the skin, in which the display unit displays the measured elastic modulus, the case stores or supports the elastic modulus measuring unit, the case is provided with an abutting member which is caused to abut on the skin, and the elastic modulus measuring unit includes a contact pressure sensor detecting the contact pressure applied to the abutting member from the skin, a deformation amount sensor detecting the deformation amount of the skin on which the abutting member is caused to abut, and an elastic modulus calculation unit calculating the elastic modulus of the skin based on the detected contact pressure and the detected deformation amount of the skin.
   According to the above-described configuration, the accumulation level measuring unit measuring the accumulation level of the fluorescent substances and the elastic modulus measuring unit measuring the elastic modulus of the skin corresponding to the elasticity of the skin are supported by the same case. Therefore, the portable measuring instrument of the above-described configuration is portable while enabling both the measurement of the accumulation level of the fluorescent substances accumulated in the skin and the measurement of the elasticity of the skin.
(3) Preferably, the case is provided with a holding member which movably supports the abutting member and which is to be held by a user and an elastic member connecting the abutting member and the holding member, in which the holding member is provided with a contact surface which is brought into contact with the skin and the deformation amount sensor is a deformation amount calculation unit calculating the deformation amount of the skin by calculating the deformation amount of the elastic member when the abutting member abuts on the skin and the contact surface contacts the skin based on the contact pressure and the elastic constant of the elastic member and subtracting the deformation amount of the elastic member from the projection distance in which the abutting member projects from the contact surface when the abutting member does not abut on the skin.
   According to the above-described configuration, the deformation amount of the skin is calculated by subtracting the deformation amount of the elastic member when the abutting member and the contact surface abut on the skin from the projection distance in which the abutting member projects from the contact surface when the abutting member does not abut on the skin. Since the deformation amount of the skin is detected without using a sensor directly detecting the deformation amount of the skin, the cost required for the sensor is reduced.
(4) Preferably, the abutting member stores the irradiation unit and the light receiving unit and the contact pressure sensor is fixed to the abutting member.
   According to the above-described configuration, the irradiation unit and the light receiving unit are stored in the abutting member and the contact pressure sensor is fixed to the abutting member. Therefore, when the abutting member abuts on the skin, the irradiation unit can irradiate the skin with excitation light, the light receiving unit can receive fluorescence, and the contact pressure sensor can detect the contact pressure.
(5) Preferably, the holding member is provided with a probe unit movably supporting the abutting member and a body unit to/from which the probe unit is attachable and detachable, in which the probe unit stores the elastic member and the body unit stores the accumulation level calculation unit, the deformation amount calculation unit, and the elastic modulus calculation unit.
   According to the above-described configuration, the display unit, the accumulation level calculation unit, the deformation amount calculation unit, and the elastic modulus calculation unit are stored in the body unit, the elastic member is stored in the probe unit, and the irradiation unit and the light receiving unit are stored in and the contact pressure sensor is fixed to the abutting member supported by the probe unit. Since the probe unit is attachable and detachable to/from the body unit, the irradiation unit, the light receiving unit, and the contact pressure sensor are easily exchanged.
(6) Preferably, the irradiation unit is an ultraviolet LED emitting the excitation light having a 370 nm peak wavelength.
   According to the above-described configuration, the excitation light having a 370 nm peak wavelength is emitted, and therefore the accumulation level of the AGEs emitting 440 nm fluorescence to the excitation light having a 370 nm wavelength is efficiently measured.

### Advantageous Effects of Invention

According to the present invention, the portable measuring instrument is portable while enabling both the measurement of the accumulation level of the fluorescent substances accumulated in the skin and the measurement of the elasticity of the skin.

### Brief Description of Drawings

Fig. 1 is a perspective view illustrating the appearance of a portable measuring instrument 10.
Fig. 2 is a perspective view illustrating the portable measuring instrument 10 in a state where a probe unit 13 is removed from a body unit 14.
Fig. 3 is a III-III cross-sectional view of Fig. 2 illustrating an abutting member 11 and the probe unit 13 in non-measurement.
Fig. 4 is a cross-sectional view schematically illustrating a sensor chip 26.
Fig. 5 is a cross-sectional view illustrating the abutting member 11 and the probe unit 13 in measurement.
Fig. 6 is a block diagram illustrating the electric configuration of the portable measuring instrument 10.

### Description of Embodiments

Hereinafter, a preferable embodiment of the present invention is described with reference to the drawings as appropriate. The embodiment described below is only an example of the present invention. It is a matter of course that the embodiment of the present invention can be altered as appropriate without changing the gist of the present invention.

The portable measuring instrument 10 is a device measuring the accumulation level of fluorescent substances per unit area in a skin 30 and the elastic modulus of the skin 30. In this embodiment, the fluorescent substances to be measured are AGEs (Advanced Glycation End Products). The measurement of the accumulation level of the AGEs is performed by irradiating the AGEs in the skin 30 with excitation light 15 to cause the AGEs to generate fluorescence 16, and then detecting the intensity of the generated fluorescence 16. The measurement of the elastic modulus of the skin 30 is performed by detecting the depression amount of the skin 30 and the contact pressure when an abutting member 11 described later is caused to abut on the skin 30.

### [Case 20 and Holding member 12]

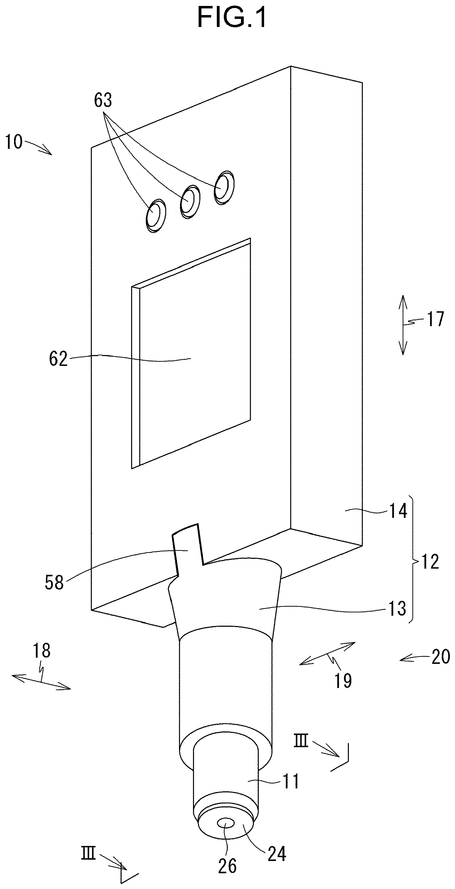

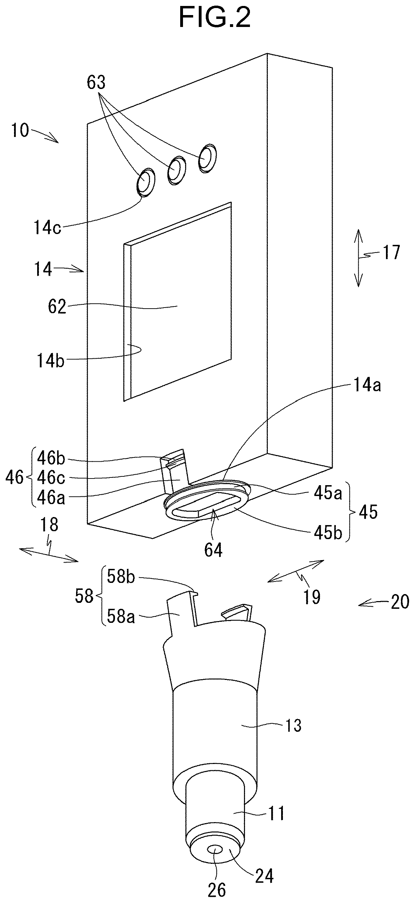

As illustrated in Fig. 1 and Fig. 2, the portable measuring instrument 10 is provided with a case 20. The case 20 is provided with the abutting member 11 which is caused to abut on the skin 30 and a holding member 12 to be held by a user. The holding member 12 is provided with a probe unit 13 movably supporting the abutting member 11 and a body unit 14 to/from which the probe unit 13 is attachable and detachable.

In the following description, a direction where the abutting member 11 and the holding member 12 are arranged is defined as a central axis direction 17 of the portable measuring instrument 10. In the central axis direction 17, the side where the abutting member 11 is present is a front end side and the side where the holding member 12 is present is a rear end side. A first radial direction 18 and a second radial direction 19 are directions orthogonal to each other and are directions orthogonal to the central axis direction 17. In particular, the first radial direction 18 is a direction where a display surface of a display unit 62 described later stored in the holding member 12 is directed.

### [Abutting member 11]

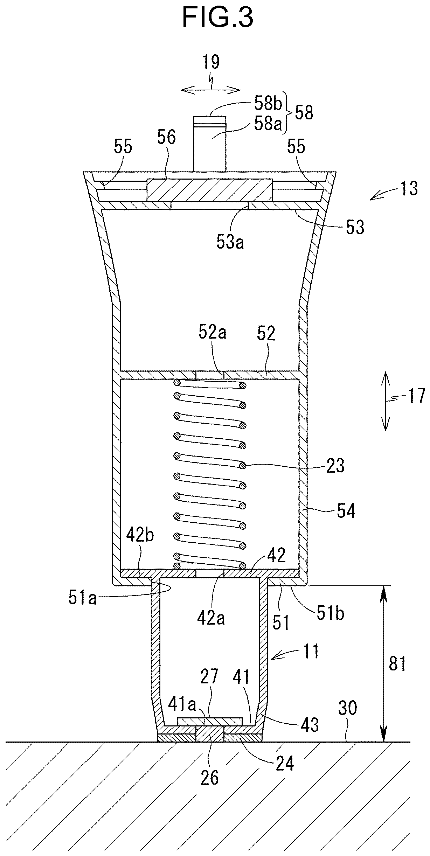

As illustrated in Fig. 1 and Fig. 3, the abutting member 11 is a hollow container having a columnar outer shape. The abutting member 11 is provided with a disk-shaped front end wall 41, a disk-shaped rear end wall 42, and a cylindrical side wall 43. The front end wall 41 and the rear end wall 42 are arranged along the central axis direction 17. The side wall 43 connects the front end wall 41 and the rear end wall 42 along the central axis direction 17. An opening portion 41a is formed in the center of the front end wall 41. A sensor chip 26 is disposed in the opening portion 41a. The sensor chip 26 is fixed to a substrate 27. The substrate 27 is fixed to the front end wall 41. A contact pressure sensor 24 is fixed to the front end surface of the front end wall 41. An opening portion 42a allowing a wiring line, such as a signal line, to pass is formed in the center of the rear end wall 42. An outer peripheral portion of the rear end wall 42 is a flange portion 42b projecting outward in the first radial direction 18 and the second radial direction 19 relative to the side wall 43.

### [Sensor chip 26]

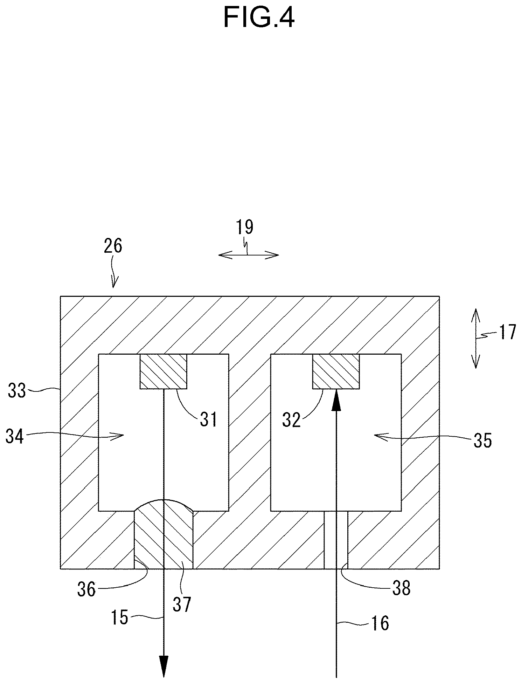

As illustrated in Fig. 4, the sensor chip 26 is provided with an ultraviolet LED (example of the irradiation unit) 31, a photodiode (example of the light receiving unit) 32, and a package 33 storing the ultraviolet LED 31 and the photodiode 32.

The package 33 is hollow inside and has a substantially rectangular parallelepiped shape. The package 33 is provided with a light emitting space 34 where the ultraviolet LED 31 is disposed and a light receiving space 35 where the photodiode 32 is disposed. The package 33 is provided with a through-hole 36 causing the light emitting space 34 to communicate with the outside, a silicon microlens 37 disposed in the through-hole 36, and a through-hole 38 causing the light receiving space 35 to communicate with the outside.

The ultraviolet LED 31 is a device emitting the excitation light 15 toward the skin 30. Herein, the AGEs are fluorescent substances generating the 440 nm fluorescence 16 when the excitation light 15 having a 370 nm wavelength is emitted thereto. Therefore, the ultraviolet LED 31 is configured to emit the excitation light 15 having a 370 nm peak wavelength so as to obtain the fluorescence 16 corresponding to the AGEs. The ultraviolet LED 31 may be able to emit the excitation light 15 in a wavelength range including 370 nm and is not limited to the case where the peak wavelength of the excitation light 15 is 370 nm.

The excitation light 15 emitted from the ultraviolet LED 31 is output to the outside of the package 33 to be emitted to the skin 30 located at the position facing the abutting member 11 through the silicon microlens 37. When the AGEs in the skin 30 are irradiated with the excitation light 15, the fluorescence 16 is generated from the AGEs.

The photodiode 32 is a device receiving the fluorescence 16 generated from the AGEs in the skin 30 irradiated with the excitation light 15 and detecting the intensity of the received fluorescence 16. Based on the intensity of the detected fluorescence 16, the accumulation level of the AGEs per unit area in the skin 30 is measured.

The sensor chip 26 or the substrate 27 may be provided with an amplification circuit amplifying an electric signal output by the photodiode 32.

### [Contact pressure sensor 24]

As illustrated in Fig. 3, the contact pressure sensor 24 is a device detecting the contact pressure applied to the abutting member 11 from the skin 30. When the abutting member 11 is pressed against the skin 30, the contact pressure sensor 24 provided on the front end surface of the abutting member 11 abuts on the skin 30. The contact pressure sensor 24 is configured by a piezoelectric element, for example, and the pressure applied to the contact pressure sensor 24 is output as an electric signal.

### [Probe unit 13]

As illustrated in Fig. 1 to Fig. 3, the probe unit 13 is a hollow container having a substantially columnar outer shape. The probe unit 13 is provided with an annular front end wall 51, a disk-shaped intermediate wall 52, a disk-shaped rear wall 53, a cylindrical side wall 54, and an annular engagement projection portion 55. The front end wall 51, the intermediate wall 52, and the rear wall 53 are arranged along the central axis direction 17. The side wall 54 connects the front end wall 51, the intermediate wall 52, the rear wall 53, and the engagement projection portion 55 along the central axis direction 17.

An opening portion 51a is formed in the center of the front end wall 51. In the opening portion 51a, the side wall 43 of the abutting member 11 is disposed. The inner diameter of the opening portion 51a is substantially in agreement with the outer shape of the side wall 43 and is smaller than the outer diameter of the flange portion 42b. Therefore, the abutting member 11 is supported without falling off from the probe unit 13. The front end surface of the front end wall 51 is a contact surface 51b which is brought into contact with the skin 30.

An opening portion 52a allowing a wiring line, such as a signal line, to pass is formed in the center of the intermediate wall 52. An opening portion 53a allowing a wiring line, such as a signal line, to pass is formed also in the center of the rear wall 53.

As illustrated in Fig. 1 and Fig. 2, the probe unit 13 is provided with two engagement portions 58. The two engagement portions 58 are arranged along the first radial direction 18 and are fixed to the side wall 54. The engagement portion 58 has an extension portion 58a extending to the rear end side and an engagement piece 58b extending radially inward from the rear end of the extension portion 58a. The engagement portions 58 can be engaged with portions to be engaged 46 described later provided in the body unit 14. The engagement projection portion 55 of the probe unit 13 can be engaged with an engagement recess portion 45 described later provided in the body unit 14. In a state where the probe unit 13 is attached to the body unit 14, the engagement portions 58 and the engagement projection portion 55 of the probe unit 13 are engaged with the portions to be engaged 46 and the engagement recess portion 45 of the body unit 14, respectively.

### [Connector 56]

As illustrated in Fig. 3, the portable measuring instrument 10 is provided with a connector 56 in the probe unit 13. The connector 56 is located on the rear end side relative to the rear wall 53 and is fixed to the rear wall 53. The connector 56 is electrically connectable to a connector 64 described later provided in the body unit 14. Signal lines from the ultraviolet LED 31, the photodiode 32, and the contact pressure sensor 24 are connected to the connector 56.

### [Elastic member 23]

As illustrated in Fig. 3, the portable measuring instrument 10 is provided with an elastic member 23 in the probe unit 13. The elastic member 23 connects the abutting member 11 and the probe unit 13 which is a part of the holding member 12. The elastic member 23 is a compression coil spring in this embodiment. The elastic member 23 abuts on the rear end wall 42 of the abutting member 11 and the intermediate wall 52 of the probe unit 13. In a state where the abutting member 11 does not contact the skin 30, the abutting member 11 is in a state of projecting to the maximum from the probe unit 13, i.e., a state where the flange portion 42b abuts on the front end wall 51 of the probe unit 13. A projection distance 81 is the distance from the contact surface 51b to the front end surface of the abutting member 11 when the abutting member 11 projects to the maximum from the probe unit 13. In the state where the abutting member 11 projects to the maximum from the probe unit 13, the elastic member 23 is in a compressed state. Therefore, the elastic member 23 always energizes the abutting member 11 to the front end side.

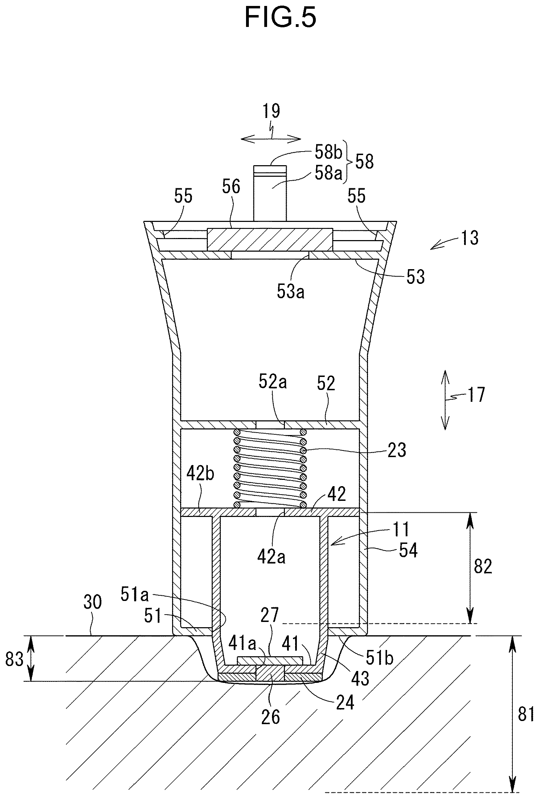

The abutting member 11 is pressed against the skin 30 until the contact surface 51b contacts the skin 30 when the elastic modulus of the skin 30 is measured as illustrated in Fig. 5. At this time, the skin 30 is depressed by the abutting member 11 and the abutting member 11 is pressed into the rear end side against the energizing force of the elastic member 23. Herein, the elastic modulus of the skin 30 is the elastic modulus when the skin 30 is considered to be an elastic body. The elastic modulus of the skin 30 is specified utilizing the Hooke's law based on the external force applied to the skin 30 when the skin 30 is depressed and the deformation amount (depression amount) 82 of the skin 30 at that time. The external force applied to the skin 30 is equal to the force applied to the abutting member 11 from the skin 30. This force is specified based on the contact pressure applied to the abutting member 11 from the skin 30. The deformation amount 82 of the skin 30 is equal to the distance obtained by subtracting the deformation amount (contraction amount) 83 of the elastic member 23 from the projection distance 81. The deformation amount 83 of the elastic member 23 is specified utilizing the Hooke's law based on the elastic modulus of the elastic member 23 and the force applied to the abutting member 11. Since the elastic modulus of the elastic member 23 is specified beforehand, an unknown is only the force applied to the abutting member 11. Therefore, when the contact pressure applied to the abutting member 11 from the skin 30 is detected, the deformation amount 82 of the skin 30 can be specified and further the elastic modulus of the skin 30 can be specified.

### [Body unit 14]

As illustrated in Fig. 1 and Fig. 2, the body unit 14 is a hollow container having an outer shape of a rectangular parallelepiped shape in this embodiment. The body unit 14 has an opening portion 14a formed on the front end side and an opening portion 14b and three opening portions 14c formed on one side in the first radial direction 18. In the body unit 14, the display unit 62, three operating units 63, and the connector 64 are fixed. The connector 64 is disposed in the opening portion 14a on the front end side. The display unit 62 is exposed from the opening portion 14b. Each of the three operating units 63 is exposed from each of the three opening portions 14c.

As illustrated in Fig. 2, the body unit 14 is provided with the engagement recess portion 45 and the two portions to be engaged 46 around the opening portion 14a formed on the front end side.

The engagement recess portion 45 is provided with a cylindrical cylinder portion 45a extending to the front end side and a flange portion 45b expanding radially outward from the front end of the cylinder portion 45a. The outer peripheral surface of the cylinder portion 45a is depressed to the outer peripheral surface of the flange portion 45b. Therefore, the engagement projection portion 55 of the probe unit 13 can be engaged with the engagement recess portion 45 of the body unit 14.

The two portions to be engaged 46 correspond to the two engagement portions 58 of the probe unit 13 and are arranged along the first radial direction 18. The portion to be engaged 46 has a first recess portion 46a, a second recess portion 46b, and an engagement piece 46c. The first recess portion 46a is formed to be depressed from the front end surface of the body unit 14 and the outer surface in the first radial direction 18. The second recess portion 46b is formed to be depressed from the outer surface in the first radial direction 18 on the rear end side of the first recess portion 46a. The engagement piece 46c is a portion extending outward in the first radial direction 18 and partitions the first recess portion 46a and the second recess portion 46b. The engagement pieces 58b of the probe unit 13 can be engaged with the second recess portions 46b of the body unit 14.

By moving the probe unit 13 in a separated state to the rear end side to the body unit 14, the probe unit 13 can be attached to the body unit 14. When the probe unit 13 is brought close to the body unit 14, the engagement pieces 58b of the probe unit 13 first enter the first recess portions 46a of the body unit 14. When the probe unit 13 further moves to the rear end side, the engagement pieces 58b of the probe unit 13 abut on the engagement pieces 46c of the body unit 14. Since the engagement portions 58 (probe unit 13) are formed of resin, the extension portions 58a of the probe unit 13 are elastically deformed with the movement, and then the engagement pieces 58b of the probe unit 13 move beyond the engagement pieces 46c of the body unit 14. As a result, the engagement pieces 58b of the probe unit 13 enter the second recess portions 46b of the body unit 14, and then the engagement portions 58 of the probe unit 13 are engaged with the portions to be engaged 46 of the body unit 14. Since the engagement projection portion 55 (probe unit 13) is formed of resin, the engagement projection portion 55 of the probe unit 13 is elastically deformed with the movement to move beyond the flange portion 45b of the body unit 14. As a result, the engagement projection portion 55 of the probe unit 13 is engaged with the engagement recess portion 45 of the body unit 14. Thus, the probe unit 13 is attached to the body unit 14. Conversely, by moving the probe unit 13 to the front end side from the body unit 14, the probe unit 13 is removed from the body unit 14 similarly utilizing the elastic deformation of the probe unit 13.

### [Display unit 62]

The display unit 62 is a device displaying the measured accumulation level of the AGEs and the measured elastic modulus of the skin 30. The display unit 62 is configured so that at least characters can be displayed and is a liquid crystal panel, for example.

### [Operating unit 63]

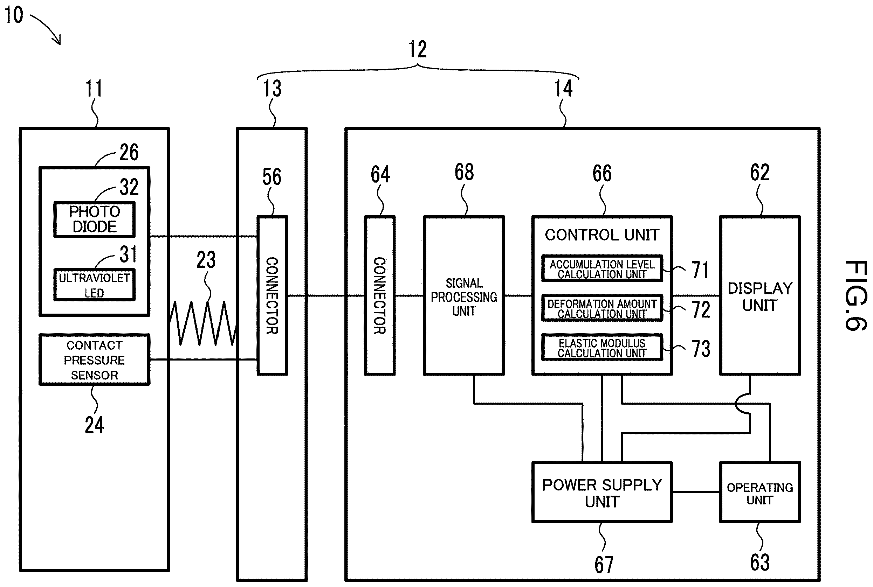

The operating unit 63 is a device for a user to input instructions into the control unit 66 (Fig. 6). The operating unit 63 is a push button, for example.

### [Connector 64]

The connector 64 is a connector electrically connectable to the connector 56 provided in the probe unit 13. The connector 64 is electrically connected to the connector 56 provided in the probe unit 13 in a state where the body unit 14 is attached to the probe unit 13 (see Fig. 1).

### [Electric configuration]

The electric configuration of the portable measuring instrument 10 is described with reference to Fig. 6. The portable measuring instrument 10 is provided with a control unit 66, a power supply unit 67, and a signal processing unit 68 in the body unit 14.

The control unit 66 is provided with a CPU (Central Processing Unit) and a memory. The memory stores various programs for controlling the operation of the portable measuring instrument 10. The control unit 66 controls the lighting of the ultraviolet LED 31 and controls the display of the display unit 62 through the signal processing unit 68 and the connectors 56 and 64 based on the instructions input from the operating unit 63. Moreover, detection signals from the photodiode 32 and the contact pressure sensor 24 are input into the control unit 66 through the connectors 56 and 64 and the signal processing unit 68.

The power supply unit 67 is a lithium ion battery, for example. The power supply unit 67 supplies power to the ultraviolet LED 31, the display unit 62, and the signal processing unit 68 based on the instructions from the control unit 66.

The signal processing unit 68 is a circuit for signal processing and includes an amplification circuit, an A/D conversion circuit, and a drive circuit of the ultraviolet LED 31, for example. The signal processing unit 68 outputs drive current. The drive current is transmitted to the ultraviolet LED 31 of the sensor chip 26 through the connectors 56 and 64. The signal processing unit 68 amplifies an electric signal received from the photodiode 32 of the sensor chip 26 through the connectors 56 and 64 and converts the same into a digital signal.

[Accumulation level calculation unit 71, Deformation amount calculation unit 72, and Elastic modulus calculation unit 73]

The control unit 66 is provided with an accumulation level calculation unit 71, a deformation amount calculation unit 72, and an elastic modulus calculation unit 73. The accumulation level calculation unit 71 calculates the accumulation level of the AGEs per unit area in the skin 30 irradiated with the excitation light 15 based on the intensity of the fluorescence 16 detected by the photodiode 32. The deformation amount calculation unit (example of the deformation amount sensor) 72 calculates the deformation amount (depression amount) 82 of the skin 30 on which the abutting member 11 is caused to abut. The deformation amount calculation unit 72 can calculate the deformation amount 82 of the skin 30 utilizing the fact that the deformation amount 82 of the skin 30 is specified based on the contact pressure detected by the contact pressure sensor 24 and the elastic modulus of the elastic member 23 as described above. Therefore, the deformation amount calculation unit 72 functions as a sensor detecting the deformation amount (depression amount) 82 of the skin 30 on which the abutting member 11 is caused to abut. The elastic modulus calculation unit 73 calculates the elastic modulus of the skin 30 based on the contact pressure detected by the contact pressure sensor 24 and the deformation amount 82 of the skin 30.

### [Accumulation level measuring unit and Elastic modulus measuring unit]

The portable measuring instrument 10 is provided with an accumulation level measuring unit measuring the accumulation level of the AGEs and an elastic modulus measuring unit measuring the elastic modulus of the skin 30 by the above-described configuration. The accumulation level measuring unit is provided with the ultraviolet LED 31, the photodiode 32, and the accumulation level calculation unit 71. The elastic modulus measuring unit is provided with the contact pressure sensor 24, the deformation amount calculation unit 72 as the deformation amount sensor, and the elastic modulus calculation unit 73.

### [Use example]

The portable measuring instrument 10 is used as follows, for example. A user holds the portable measuring instrument 10 by putting the body unit 14 which is a part of the holding member 12 between fingers. A user starts the portable measuring instrument 10 by inputting and operating the operating unit 63. The user moves the started portable measuring instrument 10 to bring the abutting member 11 close to the skin 30 of the face of the user, for example. The user presses the portable measuring instrument 10 against the skin 30 until the contact surface 51b of the probe unit 13 contacts the skin 30. As a result, the skin 30 is depressed by the abutting member 11 and the abutting member 11 is pressed into the probe unit 13. When the abutting member 11 contacts the skin 30 and further abuts on the same, the contact pressure applied to the abutting member 11 from the skin 30 is detected by the contact pressure sensor 24. An electric signal corresponding to the contact pressure is output to the control unit 66 from the contact pressure sensor 24.

When the electric signal corresponding to the contact pressure is input, the control unit 66 starts the ultraviolet LED 31 to cause the ultraviolet LED 31 to emit the excitation light 15. By the irradiation with the excitation light 15, the fluorescence 16 is generated from the AGEs in the skin 30. The fluorescence 16 is detected by the photodiode 32. An electric signal corresponding to the intensity of the fluorescence 16 is output from the photodiode 32. The electric signal is input into the control unit 66 via the signal processing unit 68. The accumulation level calculation unit 71 of the control unit 66 calculates the accumulation level of the AGEs per unit area in the skin 30 based on the electric signal corresponding to the intensity of the fluorescence 16.

When the electric signal corresponding to the contact pressure is input, the deformation amount calculation unit 72 of the control unit 66 calculates the deformation amount 82 of the skin 30 based on the electric signal. The elastic modulus calculation unit 73 of the control unit 66 calculates the elastic modulus of the skin 30 based on the detected contact pressure and the calculated deformation amount 82 of the skin 30.

The control unit 66 may calculate an evaluation result of the AGEs corresponding to the accumulation level of the AGEs in accordance with the accumulation level of the AGEs. The evaluation result of the AGEs is an estimated age corresponding to the accumulation level of the AGEs, for example. Similarly, an evaluation result of the elasticity of the skin 30 may be calculated in accordance with the elastic modulus of the skin 30. The evaluation result of the elasticity of the skin 30 is the skin age, for example.

The control unit 66 causes the display unit 62 to display the calculated accumulation level of the AGEs and the calculated elastic modulus of the skin 30. The control unit 66 may cause the display unit 62 to display the evaluation result of the accumulation level of the AGEs and the evaluation result of the elasticity of the skin 30 in place of or in addition to the accumulation level of the AGEs and the elastic modulus of the skin 30.

When the electric signal corresponding to the contact pressure disappears, the control unit 66 stops the drive of the ultraviolet LED 31. When the measurement of the accumulation level of the AGEs and the elastic modulus of the skin 30 is completed, the user stops the portable measuring instrument 10 by inputting and operating the operating unit 63.

### [Operational effects of this embodiment]

According to the portable measuring instrument 10 of this embodiment, the accumulation level measuring units (31, 32, 71) measuring the accumulation level of the fluorescent substances (AGEs) are stored in or supported by the case 20. Therefore, the portable measuring instrument 10 of the above-described configuration is portable while enabling the measurement of the accumulation level of the fluorescent substances (AGEs) accumulated in the skin 30.

Moreover, the accumulation level measuring units (31, 32, 71) measuring the accumulation level of the fluorescent substances (AGEs) and the elastic modulus measuring units (24, 72, 73) measuring the elastic modulus of the skin 30 corresponding to the elasticity of the skin 30 are supported by the same case 20. Therefore, the portable measuring instrument 10 of the above-described configuration is portable while enabling both the measurement of the accumulation level of the fluorescent substances (AGEs) accumulated in the skin 30 and the measurement of the elasticity of the skin 30.

Moreover, according to the embodiment described above, the deformation amount 82 of the skin 30 is calculated by subtracting the deformation amount 83 of the elastic member 23 when the abutting member 11 and the contact surface 51b abut on the skin 30 from the projection distance 81 in which the abutting member 11 projects from the contact surface 51b when the abutting member 11 does not abut on the skin 30. Since the deformation amount 82 of the skin 30 is detected without using the sensor directly detecting the deformation amount 82 of the skin 30, the cost required for the sensor is reduced.

Moreover, according to the embodiment described above, the ultraviolet LED 31 and the photodiode 32 are stored in the abutting member 11 and the contact pressure sensor 24 is fixed to the abutting member 11. Therefore, when the abutting member 11 abuts on the skin 30, the ultraviolet LED 31 can irradiate the skin 30 with the excitation light 15, the photodiode 32 can receive the fluorescence 16, and the contact pressure sensor 24 can detect the contact pressure.

Moreover, according to the embodiment described above, the display unit 62, the accumulation level calculation unit 71, the deformation amount calculation unit 72, and the elastic modulus calculation unit 73 are stored in the body unit 14, the elastic member 23 is stored in the probe unit 13, and the ultraviolet LED 31 and the photodiode 32 are stored in and the contact pressure sensor 24 is fixed to the abutting member 11 supported by the probe unit 13. Since the probe unit 13 is attachable and detachable to/from the body unit 14, the ultraviolet LED 31, the photodiode 32, and the contact pressure sensor 24 are easily exchanged.

Moreover, according to the embodiment described above, the excitation light 15 having a 370 nm peak wavelength is emitted, and therefore the accumulation level of the AGEs emitting the 440 nm fluorescence to the excitation light 15 having a 370 nm wavelength is efficiently measured.

### [Modification]

In the embodiment described above, the portable measuring instrument 10 is provided with the accumulation level measuring units (31, 32, 71) and the elastic modulus measuring units (24, 72, 73) and the accumulation level measuring units (31, 32, 71) and the elastic modulus measuring units (24, 72, 73) are stored in or supported by the same case 20. In place of such a configuration, the portable measuring instrument 10 may be provided with only the accumulation level measuring units (31, 32, 71) and the accumulation level measuring units (31, 32, 71) may be stored in or supported by the case 20.

In the embodiment described above, the deformation amount 82 of the skin 30 is calculating by subtracting the deformation amount 83 of the elastic member 23 from the projection distance 81 in which the abutting member 11 projects from the contact surface 51b when the abutting member 11 does not abut on the skin 30. The calculation is performed by the deformation amount calculation unit 72. In place of such a configuration, the portable measuring instrument 10 may be provided with a sensor detecting the deformation amount 83 (movement amount of the abutting member 11) of the elastic member 23 varying corresponding to the deformation amount 82 of the skin 30. Alternatively, the portable measuring instrument 10 may be provided with a sensor directly detecting the deformation amount 82 of the skin 30, e.g., a laser distance sensor.

In the embodiment described above, although the compression coil spring is used as the elastic member 23, elastic bodies, such as a sponge, a porous elastic body, and a gel elastic body, may be used.

In the embodiment described above, although the abutting member 11 stores the ultraviolet LED 31 and the photodiode 32, the position where the ultraviolet LED 31 and the photodiode 32 are disposed is not limited. The ultraviolet LED 31 and the photodiode 32 may be disposed in the probe unit 13 or the body unit 14 in place of the abutting member 11 insofar as the ultraviolet LED 31 and the photodiode 32 are disposed in the case 20.

In the embodiment described above, although the holding member 12 is provided with the probe unit 13 and the body unit 14 to/from which the probe unit 13 is attachable and detachable, the holding member 12 may be configured by one inseparable container.

### Reference Signs List

10 portable measuring instrument
11 abutting member
12 holding member
13 probe unit
14 body unit
15 excitation light
16 fluorescence
20 case
23 elastic member
24 contact pressure sensor
30 skin
31 ultraviolet LED (example of irradiation unit)
32 photodiode (example of light receiving unit)
51b contact surface
62 display unit
71 accumulation level calculation unit
72 deformation amount calculation part (example of deformation amount sensor)
73 elastic modulus calculation unit

## Claims

1. A portable measuring instrument comprising:
an accumulation level measuring unit measuring an accumulation level of a fluorescent substance accumulated per unit area in a skin;
a display unit displaying the measured accumulation level; and
a case which stores or supports the accumulation level measuring unit and the display unit and which is to be held by a user, wherein
the accumulation level measuring unit includes
an irradiation unit emitting excitation light toward the skin,
a light receiving unit receiving fluorescence generated from the fluorescent substance in the skin irradiated with the excitation light and detecting intensity of the received fluorescence, and
an accumulation level calculation unit calculating the accumulation level of the fluorescent substance based on the detected intensity.

2. The portable measuring instrument according to Claim 1 further comprising:
an elastic modulus measuring unit measuring an elastic modulus of the skin, wherein
the display unit displays the measured elastic modulus,
the case stores or supports the elastic modulus measuring unit,
the case is provided with an abutting member which is caused to abut on the skin, and
the elastic modulus measuring unit includes
a contact pressure sensor detecting a contact pressure applied to the abutting member from the skin,
a deformation amount sensor detecting a deformation amount of the skin on which the abutting member is caused to abut, and
an elastic modulus calculation unit calculating the elastic modulus of the skin based on the detected contact pressure and the deformation amount of the skin.

3. The portable measuring instrument according to Claim 2, wherein
the case includes
a holding member which movably supports the abutting member and which is to be held by a user, and
an elastic member connecting the abutting member and the holding member,
the holding member includes a contact surface which is brought into contact with the skin, and
the deformation amount sensor is a deformation amount calculation unit calculating the deformation amount of the skin by calculating a deformation amount of the elastic member when the abutting member abuts on the skin and the contact surface contacts the skin based on the contact pressure and an elastic constant of the elastic member and subtracting the deformation amount of the elastic member from a projection distance in which the abutting member projects from the contact surface when the abutting member does not abut on the skin.

4. The portable measuring instrument according to Claim 3, wherein
the abutting member stores the irradiation unit and the light receiving unit, and
the contact pressure sensor is fixed to the abutting member.

5. The portable measuring instrument according to Claim 4, wherein
the holding member includes
a probe unit movably supporting the abutting member, and
a body unit to/from which the probe unit is attachable and detachable, wherein
the probe unit stores the elastic member, and
the body unit stores the display unit, the accumulation level calculation unit, the deformation amount calculation unit, and the elastic modulus calculation unit.

6. The portable measuring instrument according to any one of Claims 1 to 5, wherein
the irradiation unit is an ultraviolet LED emitting the excitation light having a 370 nm peak wavelength.
